**Europäisches Patentamt**

**European Patent Office**

**Office européen des brevets**

(19)

(11) Numéro de publication : **0 329 512 B1**

(12) **FASCICULE DE BREVET EUROPEEN**

(45) Date de publication du fascicule du brevet :
06.05.92 Bulletin 92/19

(21) Numéro de dépôt : **89400293.0**

(22) Date de dépôt : **02.02.89**

(51) Int. Cl.⁵ : **C07C 219/08,** C07C 237/50

(51) Int. Cl.⁵ : **C07C 219/08,** C07C 237/50

(54) **Procédé de quaternisation.**

(30) Priorité : **11.02.88 FR 8801635**

(43) Date de publication de la demande :
**23.08.89 Bulletin 89/34**

(45) Mention de la délivrance du brevet :
**06.05.92 Bulletin 92/19**

(84) Etats contractants désignés :
**AT BE CH DE ES FR GB GR IT LI LU NL SE**

(56) Documents cités :
**EP-A- 0 250 325**

(73) Titulaire : **ATOCHEM
4 & 8, Cours Michelet La Défense 10
F-92800 Puteaux (FR)**

(72) Inventeur : **Lacroix, Christian
11, Rue de l'Eglise - Forkling
F-57600 Forbach (FR)**
Inventeur : **Hess, Raymond
21, Rue Lepinseck
F-57600 Forbach (FR)**

(74) Mandataire : **Rieux, Michel et al
ATOCHEM Département Propriété Industrielle
4, Cours Michelet - La Défense 10 - Cedex 42
F-92091 Paris-La Défense (FR)**

Jouve, 18, rue Saint-Denis, 75001 PARIS

## Description

La présente invention a pour objet un procédé de préparation de solutions aqueuses de sels insaturés d'ammonium quaternaire répondant à la formule (I) suivante :

$H_2C = C(R_3) - C(O) - A - R_4 - N^+(R_1)(R_2)(R), X^-$

dans laquelle :

– A est un atome d'oxygène ou un groupe NH,

– $R_3$ est un atome d'hydrogène ou un radical méthyle,

– $R_4$ est un radical alkyle, linéaire ou ramifié de 1 à 6 atomes de carbone,

– $R_1$, $R_2$ et R, différents ou identiques, sont un radical alkyle ou un radical aryle,

– X est choisi parmi Cl, Br, I, $CH_3 - CO_3$ ou $CH_3 - SO_4$.

Il est connu et décrit dans la demande européenne 250 325 un procédé de préparation de solutions aqueuses de sels insaturés d'ammonium quaternaire (I) selon lequel, en présence d'au moins un inhibiteur de polymérisation :

– on fait réagir, dans une première étape (a) au moins un monomère (méth)acrylique (II) de formule $H_2C = C(R_3) - C(O) - A - R_4 -N(R_1)(R_2)$ (dans laquelle $R_1$, $R_2$, $R_3$, $R_4$ et A ont la signification mentionnée ci-dessus) avec 5 à 20 % en poids de la quantité nécessaire à la réaction d'agent quaternisant (III) de formule RX (avec R et X ayant la signification précitée), ou, à la place de RX, avec 5 à 20 % en poids d'une solution aqueuse de sels insaturés d'ammonium quaternaire (I) (donné par rapport au poids de monomères (méth)acryliques (II)), cette solution comprenant 50 à 85 % en poids de sels d'ammoniums quaternaires (I),

– dans une deuxième étape (b), on ajoute en continu l'eau et l'agent quaternisant (III) jusqu'à l'obtention de la concentration souhaitée de sels insaturés d'ammoniums quaternaires dans l'eau.

Pendant les étapes (a) et (b), la température est maintenue entre 30 et 60°C. De plus, pendant les étapes (a) et (b) et en particulier à l'approche de la fin de la réaction, on maintient dans le milieu réactionnel un courant de gaz oxygéné tel que le rapport en volume (ou débit volumétrique) de gaz total à la sortie du réacteur sur le volume (ou débit volumétrique) d'oxygène introduit à l'entrée de ce même réacteur est inférieur à 100.

Le procédé selon la demande européenne 250 325 permet de préparer des solutions aqueuses de sels d'ammonium quaternaire insaturés (I) qui ont une stabilité à température ambiante supérieure à un an. Toutefois, on constate dans ces solutions la présence d'impuretés, en particulier de $CH_2 = C(R_3) - C(O) - A - R$ (IV), $CH_2 = C(R_3) - C(O) - AH$ (V) et de monomères (méth)acryliques (II). La teneur de ces impuretés est particulièrement élevée lorsque $R_3$ est un atome d'hydrogène et que A représente un atome d'oxygène : elle peut alors atteindre respectivement des valeurs de 3000 - 5000 ppm, 0,69 % et 1,3 % en poids pour les impuretés (IV), (V) et (II).

La demanderesse a découvert un procédé de préparation de solutions aqueuses de sels insaturés d'ammonium quaternaire (I) permettant de réduire considérablement la formation des impuretés lors de la réaction de quaternisation, ce procédé ayant également l'avantage de donner des solutions dont la stabilité à température ambiante excède un an.

Plus précisément, la présente invention a pour objet un procédé de préparation de solutions aqueuses de sels insaturés d'ammonium quaternaire (I) répondant à la formule suivante :

$H_2C = C(R_3) - C(O) - A - R_4 - N^+(R_1)(R_2)(R), X^-$,

à partir d'au moins un monomère (méth)acrylique (II) de formule $H_2C = C(R_3) - C(O) - A - R_4 - N(R_1)(R_2)$ et d'au moins un agent quaternisant (III) de formule RX, dans lesquelles R, $R_1$, $R_2$, $R_4$, A et X ont la signification précitée, en présence d'au moins un inhibiteur de polymérisation, procédé caractérisé en ce que la réaction est effectuée à une température comprise entre 10°C et 80°C, en ce que,

(a) dans une première étape, on introduit dans le réacteur la totalité ou une partie de l'agent quaternisant (III) nécessaire à la réaction, cet agent (II) étant à l'état liquide dans les conditions de la réaction,

(b) ensuite, on ajoute au moins un monomère (méth)acrylique (II), et

(c) dès que 0 à 30 % de la stoechiométrie du ou des monomères (méth)acryliques ont été introduits dans le réacteur, on ajoute en continu et simultanément le reste d'agent quaternisant (III), le reste de monomères (méth)acryliques et l'eau jusqu'à l'obtention de la concentration souhaitée de sels insaturés d'ammonium quaternaire (I),

(d) et, dans le cas où l'agent quaternisant (III) est à l'état gazeux à la température de réaction, la réaction est effectuée en présence d'oxygène et on impose une pression de manière à ce que l'agent quaternisant soit à l'état liquide à la température de réaction, et, en fin de réaction, on diminue progressivement la pression jusqu'à la pression atmosphérique et simultanément on impose un rapport en débit volumétrique de gaz total à la sortie du réacteur sur le débit volumétrique d'oxygène introduit dans le réacteur inférieur à 100, et de préférence inférieur à 50.

De préférence, la température est maintenue entre 30 et 60°C pendant la réaction.

De préférence, pendant l'étape (c), le débit d'eau, des monomères (méth)acryliques (II) et éventuellement d'agent quaternisant (III) sont réglés de manière à maintenir dans le milieu réactionnel une solution saturée ou proche de la saturation en sels insaturés d'ammonium quaternaire (I).

De préférence, l'introduction du reste d'agent quaternisant (III), du reste de monomères (méth)acryliques (II) et d'eau dans l'étape (c) est effectuée dès que 10 à 20 % de la stoechiométrie du ou des monomères (méth)acryliques ont été introduits dans le réacteur conformément à l'étape (b).

Dans le cas ou l'agent quaternisant est à l'état liquide à la température de réaction, la réaction peut être effectuée en présence d'oxygène sous pression réduite ou sous pression. De préférence, toutefois, dans ce cas précis on impose la pression atmosphérique et la réaction est éventuellement effectuée en présence d'oxygène.

Dès que la réaction est terminée, les traces d'agents quaternisants (III) volatils dissous dans le mélange réactionnel sont éliminés par un balayage du mélange sous pression réduite ou à pression atmosphérique à l'aide d'un gaz oxygéné tel que de l'air ou de l'oxygène pur.

Le procédé selon l'invention convient notamment à la préparation de solutions aqueuses comprenant des mélanges de sels insaturés d'ammonium quaternaire (I) (ci-après denommées solutions de sels mixtes). Si les agents quaternisants (III) nécessaires à la préparation de ces solutions de sels mixtes sont pour les uns gazeux à la température de réaction, et pour les autres liquides à la température de réaction, il est recommandé d'introduire en premier les agents quaternisants (III) liquides et ensuite seulement les agents quaternisants (III) gazeux. De préférence, on procède alors de la manière suivante :

– La réaction est effectuée en présence d'au moins un inhibiteur de polymérisation entre au moins un monomère (méth)acrylique (II) et au moins un agent quaternisant (III) et elle est caractérisée en ce que elle est effectuée à une température comprise entre 10°C et 80°C, et de préférence entre 30 et 60°C,
en ce que,
($a_1$) dans une première étape, on introduit dans le réacteur la totalité ou une partie des ou de l'agent quaternisant (III) liquide,
($b_1$) ensuite, on ajoute au moins un monomère (méth)acrylique (II), et
($c_1$) dès que 0 à 30 % de la stoechiométrie du ou des monomères (méth)acryliques ont été introduits dans le réacteur, on ajoute en continu et simultanément le reste d'agent quaternisant (III) liquide à la température de réaction, la totalité ou une partie du reste de monomères (méth)acryliques (II) nécessaires à la réaction et la totalité ou une partie de l'eau nécessaire à la réaction, la quantité totale dudit agent quaternisant (III) liquide à la température de réaction représentant 0,1 % à 99, 9 % de la stoechiométrie par rapport aux dits monomères (méth)acryliques (II),
($d_1$) et ensuite, on ajoute en continu et simultanément les ou l'agent quaternisant (III) gazeux à la température de réaction, le reste de monomères (méth)acryliques (II) et le reste d'eau jusqu'à l'obtention de la concentration souhaitée de sels insaturés d'ammonium quaternairé (I), la somme des quantités d'agents quaternisants ajoutés dans les étapes ($a_1$), ($c_1$) et ($d_1$) correspondant à une valeur égale ou supérieure à la stoechiométrie par rapport aux monomères (méth)acryliques (II), la réaction étant alors effectuée en présence d'oxygène à pression atmosphérique ou à une pression supérieure, et en ce que, pendant la réaction, en fin de réaction, et pendant la mise à la pression atmosphérique on impose un rapport en débit volumétrique de gaz total à la sortie du réacteur sur le débit volumétrique d'oxygène introduit dans le réacteur inférieur à 100, et de préférence inférieur à 50.

L'étape ($a_1$) peut être effectuée sous pression ou sous une pression réduite. De préférence, elle est effectuée sous la pression atmosphérique.

Le procédé selon l'invention convient à la quaternisation des monomères (méth)acryliques (II) susceptibles de s'hydrolyser tels que l'acrylate de diméthylaminoéthyle, l'acrylate de diméthylaminopropyle, et les méthacrylates correspondants, ainsi qu'au diméthylaminopropyle acrylamide et au diméthylaminopropyle méthacrylamide.

Les agents quaternisants (III) convenant bien à la présente invention sont notamment les hydrocarbures halogènés. Parmi les agents quaternisants (III) liquides dans les conditions normales de température et de pression, on peut citer l'iodure de méthyle, le bromure d'éthyle, l'iodure d'éthyle, le chlorure de benzyle conviennent également les sulfate de diméthyle et le carbonate de diméthyle. Parmi les agents quaternisants (III) gazeux dans les conditions normales de température et de pression, on peut citer le chlorure de méthyle, le bromure de méthyle, le chlorure d'éthyle.

Parmi les inhibiteurs de polymérisation convenant au procédé selon l'invention, on peut citer le toluène -3,5 - diterbutyl - 4 - hydroxy, l'éther méthylique d'hydroquinone, la phénothiazine, l'hydroquinone, le catéchol et le terbutylcatéchol. De préférence, on utilise de 100 ppm à 5000 ppm d'inhibiteur de polymérisation par rapport au monomère (méth)acrylique (II).

Le procédé selon l'invention permet de préparer des solutions aqueuses de sels insaturés d'ammonium quaternaire (I) ayant des concentrations de l'ordre de 50 à 85 % en poids de sels (I) dans l'eau. De plus, ces solutions aqueuses contiennent des quantités très faibles d'impuretés qui n'excèdent pas 200 ppm en $CH_2 = C(R_3) - C(O) - A - R$, 0,4 % en poids de $CH_2 = C(R_3) - C(O) - AH$ et 0,5 % en poids de monomères (méth)acryliques (II) (avec R, $R_3$ et A ayant la signification donnée précédemment).

Les exemples qui vont suivre, donnés à titre indicatif, permettront de mieux comprendre l'invention. Dans ces exemples, les pourcentages sont exprimés en % en poids.

## Exemple 1 (comparatif)

Préparation d'une solution aqueuse de chlorure d'acryloyloxyéthyl triméthyl ammonium à 80 %.

Dans un réacteur à double enveloppe, on charge sous agitation 515 d'acrylate de diméthylaminoéthyle stabilisé à l'aide de 700 ppm d'éther méthylique d'hydroquinone.

Pendant toute la durée de la réaction, on injecte en continu dans le réacteur 0,2 Nl/h d'air et on maintient :
- la température à 47°C
- la pression atmosphérique
- le débit de sortie des évents inférieur à 0,7 Nl/h (soit un rapport des débits volumétriques des évents sur l'oxygène introduit dans le réacteur inférieur à 17,5).

Dans la première étape, on injecte dans le réacteur 18 g de chlorure de méthyle ($CH_3Cl$) avec un débit de 30 g/h (soit 11 % de la quantité totale de $CH_3Cl$ nécessaire à la réaction), puis dans une seconde étape, on injecte simultanément et en continu le chlorure de méthyle et l'eau dans un rapport pondéral eau/$CH_3Cl$ compris entre 0,9 et 1,0 (d'où un rapport molaire eau/$CH_3Cl$ compris entre 2,5 et 2,8).

A l'approche de la fin de la réaction, le débit de chlorure de méthyle est progressivement réduit à 10 g/h et le débit de sortie des évents est maintenu inférieur à 1 Nl/h (d'où un rapport des débits volumétriques des évents sur l'oxygène à l'entrée du réacteur inférieur à 25).

L'opération est arrêtée après 8 heures de réaction.

Dans cette opération, on a utilisé 174 g d'eau et 196 g de chlorure de méthyle et on récupère 865 g de chlorure d'acryloyloxyéthyl triméthyl ammonium à 80 % dans l'eau.

Le produit final est ensuite soumis à une injection d'air avec un débit de 7 Nl/h pendant 1/2 heure à chaud puis pendant 1/2 heure à température ambiante.

Le produit final obtenu a les caractéristiques suivantes :

eau : 20,4 %
acide acrylique : 0,69 %
acrylate de diméthylaminoéthyle : 1,3 %
chlorure de méthyle : 15 ppm
polymère : néant
stabilité au stockage : supérieur à 1 an.

## Exemple 2

Préparation d'une solution aqueuse de chlorure d'acryloyloxyéthyl triméthyl ammonium à 80 %.

Dans un réacteur à double enveloppe, on charge sous agitation 78 g de chlorure de méthyle liquide. On porte la température à 47°C, et la pression du système s'équilibre à 9,6 bars absolus.

Dans la première étape, on injecte dans le réacteur 33 g d'acrylate de diméthylaminoéthyle stabilisé à l'aide de 700 ppm d'éther méthylique d'hydroquinone sur une durée de 20 minutes (soit 16 % de la quantité totale d'acrylate nécessaire à la réaction).

Dans une seconde étape on injecte simultanément et en continu le complément de l'acrylate et l'eau dans un rapport pondéral eau:acrylate de l'ordre de 0,4 sur une période de 2 heures.

Tout au long de la réaction la température est maintenue à 47°C et la pression dans le réacteur chute jusqu'à 3,2 bars absolus en fin d'injection des réactifs. On injecte ensuite dans le réacteur en continu 0,5 NL/h d'air et sur une période de 1 heure on ramène progressivement la pression du réacteur à la pression atmosphérique tout en conservant un débit de sortie des évents inférieur à 1,5 Nl/h (d'où un rapport des débits volumétriques des évents sur l'oxygène introduit inférieur à 15).

Dans cette opération, on a utilisé 71 g d'eau et 205 g d'acrylate de diméthylaminoéthyle et on récupère 343 g de chlorure d'acryloyloxyéthyl triméthyl ammonium à 80 % dans l'eau.

Le produit final est ensuite soumis à une injection d'air avec un débit de 7 Nl/h pendant 1/2 heure à chaud puis pendant 1/2 heure à température ambiante.

Le produit final obtenu a les caractéristiques suivantes :

eau : 20,3 %
acide acrylique : 0,23 %
acrylate de diméthylaminoéthyle : 0,15 %
chlorure de méthyle : 10 ppm
polymère : néant
stabilité au stockage : supérieur à 1 an.

## Exemple 3 comparatif

Préparation d'une solution aqueuse de chlorure d'acryloyloxyéthyl benzyl diméthyl ammonium à 80 %.

Dans un réacteur à double enveloppe, on charge sous agitation 429 g d'acrylate de diméthylaminoéthyle stabilisé à l'aide de 700 ppm d'éther d'hydroquinone.

Pendant toute la durée de la réaction, soit au total 4 heures, on maintient la température à 50°C et un débit d'air continu de 0,2 Nl/h. On introduit dans le réacteur le chlorure de benzyle à un débit de 80 g/h pendant 45 minutes soit 15,8 % de la quantité totale de chlorure nécessaire à la réaction. Puis on introduit simultanément et en continu le complément de chlorure de benzyle et l'eau à des débits respectifs de 110 g/h et 65 g/h.

Dans cette opération, on a utilisé 202 g d'eau et 380 g de chlorure de benzyle et on récupère 1010 g de chlorure d'acryloyloxyéthyl benzyl diméthyl ammonium à 80 % dans l'eau.

Le produit final obtenu a les caractéristique suivantes :

eau : 20,1 %

acide acrylique : 0,50 %

acrylate de diméthylaminoéthyle : 0,60 %

acrylate de benzyle : 2200 ppm

polymère : néant

stabilité au stockage : supérieur à 1 an.

## Exemple 4

Préparation d'une solution aqueuse de chlorure d'acryloyloxyéthyl benzyl diméthyl ammonium à 80 %.

Dans un réacteur à double enveloppe, on charge sous agitation 380 g de chlorure de benzyle.

Pendant toute la durée de la réaction, soit au total 4 heures, on maintient la température à 50°C et un débit d'air continu de 0,2 Nl/h. On introduit dans le réacteur l'acrylate de diméthylaminoéthyle (stabilisé à l'aide de 700 ppm d'éther méthylique d'hydroquinone) à un débit de 100 g/h pendant 45 minutes soit 17,5 % de la quantité totale d'acrylate nécessaire à la réaction. Puis on introduit simultanément et en continu le complément d'acrylate de diméthylaminoéthyle et l'eau à des débits respectifs de 120 g/h et 65 g/h.

Dans cette opération, on a utilisé 202 g d'eau et 429 g d'acrylate de diméthylaminoéthyle et on récupère 1010 g de chlorure d'acryloyloxéthyl benzyl diméthyl ammonium à 80 % dans l'eau.

Le produit final obtenu à les caractéristiques suivantes :

eau : 20,1 %

acide acrylique : 0,25 %

acrylate de diméthylaminoéthyle : 0,21 %

acrylate de benzyle : 200 ppm

polymère : néant

stabilité au stockage : supérieur à 1 an.

## Exemple 5

Préparation d'une solution aqueuse d'un mélange de chlorure d'acryloyloxyéthyl benzyl diméthyl ammonium et de chlorure d'acryloyloxyéthyl triméthyl ammonium à 80 %.

Dans un réacteur à double enveloppe, on charge sous agitation 360 g de chlorure de benzyle.

Pendant toute la durée de la réaction, soit au total 5 heures, on maintient la température à 50°C et un débit d'air continu de 0,2 Nl/h.

On introduit dans le réacteur l'acrylate de diméthylaminoéthyle (stabilisé à l'aide de 700 ppm d'éther méthylique d'hydroquinone) à un débit de 100 g/h pendant 45 minutes soit 17,5 % de la quantité totale d'acrylate nécessaire à la réaction. Puis on introduit simultanément et en continu le complément d'acrylate de diméthylaminoéthyle et l'eau à des débits respectifs de 120 g/h et 60 g/h.

Après 3,5 heures de réaction on injecte dans le réacteur du chlorure de méthyle avec un débit de 15 g/h pendant 1,5 heure tout en maintenant le débit de sortie des évents inférieur à 1 Nl/h (d'où un rapport des débits volumétriques des évents sur l'oxygène introduit inférieur à 25).

Dans cette opération on a utilisé au total 200 g d'eau, 429 g d'acrylate de diméthylaminoéthyle et 22,5 g de chlorure de méthyle. On récupère 997 g de solution aqueuse d'un mélange de 2 sels d'ammonium quaternaire à 80 % dans l'eau.

Le produit final est ensuite soumis à une injection d'air avec un débit de 7 Nl/h pendant 1/2 heure à chaud et 1/2 heure à température ambiante.

Le produit final obtenu à les caractéristiques suivantes :

eau : 20,2 %

acide acrylique : 0,29 %

acrylate de diméthylaminoéthyle : 0,28 %

acrylate de benzyle : 150 ppm

chlorure de benzyle : 10 ppm

chlorure de méthyle : 10 ppm

polymère : néant

stabilité au stockage : supérieur à 1 an.

## Revendications

1. Procédé de préparation de solutions aqueuses de sels insaturés d'ammonium quaternaire répondant à la formule (I) suivante :

$$H_2C = C(R_3) - C(O) - A - R_4 - N^+(R_1)(R_2)(R), X^-$$

dans laquelle :

– A est un atome d'oxygène ou un groupe NH,

– $R_3$ est un atome d'hydrogène ou un radical méthyle,

– $R_4$ est un radical alkyle, linéaire ou ramifié de 1 à 6 atomes de carbone,

– $R_1$, $R_2$ et R, différents ou identiques, sont un radical alkyle ou un radical aryle,

– X est choisi parmi Cl, Br, I, $CH_3 - CO_3$ ou $CH_3 - SO_4$, à partir d'au moins un monomère (méth)acrylique (II) de formule $H_2C = C(R_3) - C(O) - A - R_4 - N(R_1)(R_2)$ et d'au moins un agent quaternisant (III) de formule RX, dans lesquelles R, $R_1$, $R_2$, $R_3$, $R_4$, A et X ont la signification précitée, en présence d'au moins un inhibiteur de polymérisation, procédé caractérisé en ce que la réaction est effectuée à une température comprise entre 10°C et 80°C, en ce que,

(a) dans une première étape, on introduit dans le réacteur la totalité ou une partie de l'agent quaternisant (III) nécessaire à la réaction, cet agent (III) étant à l'état liquide dans les conditions de la réaction,

(b) ensuite, on ajoute au moins un monomère (méth)acrylique (II), et

(c) dès que 0 à 30 % de la stoechiométrie du ou des monomères (méth)acryliques ont été introduits dans le réacteur, on ajoute en continu et simultanément le reste d'agent quaternisant (III), le reste de monomères (méth)acryliques et l'eau jusqu'à l'obtention de la concentration souhaitée de sels insaturés d'ammonium quaternaire (I),

(d) et, dans le cas où l'agent quaternisant (III) est à l'état gazeux à la température de réaction, la réaction est effectuée en présence d'oxygène et on impose une pression de manière à ce que l'agent quaternisant soit à l'état liquide, et, en fin de réaction, on diminue progressivement la pression jusqu'à la pression atmosphérique et simultanément on impose un rapport en débit volumétrique de gaz total à la sortie du réacteur sur le débit volumétrique d'oxygène introduit dans le réacteur inférieur à 100.

2. Procédé selon la revendication 1, caractérisé en ce que, dans le cas (d) on impose un rapport en débit volumétrique de gaz total à la sortie du réacteur sur le débit volumétrique d'oxygène introduit dans le réacteur inférieur à 50.

3. Procédé selon la revendication 1 ou 2, caractérisé en ce que la température est maintenue entre 30 et 60°C pendant la réaction.

4. Procédé selon l'une quelconque des revendications 1 à 3, caractérisé en ce que l'introduction du reste d'agent quaternisant (III), du reste de monomères (méth)acryliques (II) et d'eau dans l'étape (c) est effectuée dès que 10 à 20 % de la stoechiométrie du ou des monomères (méth)acryliques ont été introduits conformément à l'étape (b).

5. Procédé selon la revendication 1 caractérisé en ce que la réaction est effectuée à une température comprise entre 10 et 80°C, et de préférence entre 30 et 60°C, en ce que :

($a_1$) dans une première étape, on introduit dans le réacteur la totalité ou une partie des ou de l'agent quaternisant (III) liquide,

($b_1$) ensuite, on ajoute au moins un monomère (méth)acrylique (II), et

($c_1$) dès que 0 à 30 % de la stoechiométrie du ou des monomères (méth)acryliques ont été introduits dans le réacteur, on ajoute en continu et simultanément le reste d'agent quaternisant (III) liquide à la température de réaction, la totalité ou une partie du reste de monomères (méth)acryliques (II) nécessaires à la réaction et la totalité ou une partie de l'eau nécessaire à la réaction, la quantité totale dudit agent quaternisant (III) liquide à la température de réaction représentant 0,1 % à 99, 9 % de la stoechiométrie par rapport aux dits monomères (méth)acryliques (II),

($d_1$) et ensuite, on ajoute en continu et simultanément les ou l'agent quaternisant (III) gazeux à la température de réaction, le reste de monomères (méth)acryliques (II) et le reste d'eau jusqu'à l'obtention de la concentration souhaitée de sels insaturés d'ammonium quaternaire (I), la somme des quantités d'agents quaternisants ajoutés dans les étapes ($a_1$), ($c_1$) et ($d_1$) correspondant à une valeur égale ou supérieure à la stoechiométrie par rapport aux monomères (méth)acryliques (II), la réaction étant alors effectuée en présence d'oxygène, à la pression atmosphérique ou sous une pression supérieure, et en ce que, pendant la réaction, en fin de réaction et pendant la mise à la pression atmosphérique, on impose un rapport en débit volumétrique de gaz total à la sortie du réacteur sur le débit volumétrique d'oxygène introduit dans le réacteur inférieur à 100, et de préférence inférieur à 50.

**Patentansprüche**

1. Verfahren zur Herstellung von wäßrigen Lösungen ungesättigter quartärer Ammoniumsalze entsprechend der nachstehenden Formel (I):

$$H_2C = C(R_3) - C(O) - A - R_4 - N+(R_1) (R_2) (R), X^-$$

worin:

– A ein Sauerstoffatom oder eine NH-Gruppe ist,

– $R_3$ ein Wasserstoffatom oder ein Methytrest ist,

– $R_4$ ein linearer oder verzweigter Alkylrest mit 1 bis 6 Kohlenstoffatomen ist,

– $R_1$, $R_2$ und R, verschieden oder identisch, ein Alkylrest oder ein Arylrest sind,

– X ausgewählt ist aus der Gruppe bestehend aus Cl, Br, J, $CH_3 - CO_3$ und $CH_3 - SO_4$, ausgehend von mindestens einem (Meth)acryl-Monomer (II) der Formel $H_2C = C(R_3) -C(O) -A - R_4 - N(R_1)(R_2)$ und mindestens einem Quaternisierungsmittel (III) der Formel RX, in welchen Formeln R, $R_1$, $R_2$, $R_3$, $R_4$, A und X die oben angegebene Bedeutung haben, in Gegenwart mindestens eines Polymerisationsinhibitors, wobei das Verfahren dadurch gekennzeichnet ist, daß die Reaktion bei einer Temperatur zwischen 10°C und 80°C durchgeführt wird, daß

(a) in einem ersten Schritt die Gesamtheit oder ein Teil des für die Reaktion erforderlichen Quaternisierungsmittels (III) in den Reaktor eingebracht wird, wobei dieses Mittel (III) unter den Reaktionsbedingungen flüssig ist,
(b) danach mindestens ein (Meth)acryl-Monomer (II) und
(c) sobald 0 bis 30°% der stöchiometrischen Menge des oder der (Meth)acryl-Monomer(s)(e) in den Reaktor eingebracht worden sind, kontinuierlich und gleichzeitig der Rest des Quaternisierungsmittels (III), der Rest der (Meth)acryl-Monomere und das Wasser bis zum Erhalt der gewünschten Konzentration der ungesättigten quartären Ammoniumsalze (I) zugegeben werden,
(d) und, im Falte, wo das Quaternisierungsmittel (III) sich bei der Reaktionstemperatur im gasförmigen Zustand befindet, die Reaktion in Gegenwart von Sauerstoff durchgeführt wird und ein solcher Druck eingestellt wird, daß sich das Quaternisierungsmittel im flüssigen Zustand befindet, und, am Ende der Reaktion, der Druck allmählich bis auf Atmosphärendruck abgesenkt und gleichzeitig ein Verhältnis des volumetrischen Durchsatzes des gesamten Gases am Ausgang des Reaktors zum volumetrischen Durchsatz des in den Reaktor eingeleiteten Sauerstoffs von weniger als 100 eingestellt wird.

2. Verfahren nach Anspruch 1, dadurch gekennzeichnet, daß im Falle (d) ein Verhältnis des volumetrischen Durchsatzes des gesamten Gares am Ausgang des Reaktors zum volumetrischen Durchsatz des in den Reaktor eingeleiteten Sauerstoffs von weniger als 50 eingestellt wird.

3. Verfahren nach Anspruch 1 oder 2, dadurch gekennzeichnet, daß die Temperatur während der Reaktion zwischen 30°C und 60°C gehalten wird,

4. Verfahren nach irgendeinem der Ansprüche 1 bis 3, dadurch gekennzeichnet, daß das Einbringen des Rests des Quaternisierungsmittels (III), des Rests der (Meth)acryl-Monomere (II) und von Wasser im Schritt (c) erfolgt, sobald 10 bis 20% der stöchiometrischen Menge des oder der (Meth)acryl-Monomer(s)(e) gemäß Schritt (b) eingebracht worden sind.

5. Verfahren nach Anspruch 1, dadurch gekennzeichnet, daß die Reaktion bei einer Temperatur zwischen 10°C und 80°C und vorzugsweise zwischen 30°C und 60°C durchgeführt wird, daß

($a_1$) in einem ersten Schritt die Gesamtheit oder ein Teil der oder des flüssigen Quaternisierungsmittel(s) (III) in den Reaktor eingebracht wird,

($b_1$) danach mindestens ein (Meth)acryl-Monomer (II) und ($c_1$) sobald 0 bis 30°% der stöchiometrischen Menge des oder der (Meth)acryl-Monomer(s)(e) in den Reaktor eingebracht worden sind, kontinuierlich und gleichzeitig der Rest des bei der Reaktionstemperatur flüssigen Quaternisierungsmittels (III), die Gesamtheit oder ein Teil des Rests der Tür die Reaktion notwendigen (Meth)acryl-Monomere und die Gesamtheit oder ein Teil des für die Reaktion notwendigen Wassers zugegeben werden, wobei die Gesamtmenge des bei der Reaktionstemperatur flüssigen Quaternisierungsmittels (III) 0,1% bis 99,9% der stöchiometrischen Menge in bezug auf die genannten (Meth)acryl-Monomere (II) ausmacht,

($d_1$) und sodann kontinuierlich und gleichzeitig die oder das bei der Reaktionstemperatur gasförmige(n) Quaternisierungsmittel (III), der Rest der (Meth)acryl-Monomere (II) und der Rest des Wassers bis zum Erhalt der gewünschten Konzentration der ungesättigten quartären Ammoniumsalze (I) zugegeben werden, wobei die Summe der in den Schritten ($a_1$), ($c_1$) und ($d_1$) zugegebenen Quaternisierungsmittelmengen einem Wert entspricht, der gleich der oder größer als die stöchiometrische Menge in bezug auf die (Meth)acryl-Monomere (II) ist, und wobei die Reaktion dann in Gegenwart von Sauerstoff, bei Atmosphärendruck oder unter einem höheren Druck durchgeführt wird, und daß während der Reaktion, am Ende der Reaktion und während der Anwendung des Atmosphärendrucks ein Verhältnis des volumetrischen Durchsatzes des gesamten Gases am Ausgang des Reaktors zum volumetrischen Durchsatz des in den Reaktor eingeleiteten Sauerstoffs von weniger als 100, vorzugsweise von weniger als 50, eingestellt wird.

**Claims**

1. Process for the preparation of aqueous solutions of unsaturated quaternary ammonium salts corresponding to the following formula (I):
$H_1C=C(R_3)-C(O)-A-R_4-N^+(R_1)(R_2)(R).X^-$
in which:
 – A is an oxygen atom or an NH group,
 – $R_3$ is a hydrogen atom or a methyl radical,
 – $R_4$ is a linear or branched alkyl radical with 1 to 6 carbon atoms,
 – $R_1$, $R_2$ and R, which are different or identical, are an alkyl radical or an aryl radical,
 – X is chosen from Cl, Br, I, $CH_3-CO_3$ or $CH_3-SO_4$, from at least one (meth)acrylic monomer (II) of formula $H_2C=C(R_3)-C(O)-A-R_4-N(R_1)(R_2)$ and from at least one quaternising agent (III) of formula RX, in which formulae R, $R_1$, $R_2$, $R_3$, $R_4$, A and X have the above-mentioned meaning, in the presence of at least one polymerisation inhibitor, process characterised in that the reaction is carried out at a temperature of between 10°C and 80°C, and in that
(a) in a first stage, all or a part of the quaternising agent (III) necessary for the reaction is introduced into the reactor, this agent (III) being in the liquid state in the reaction conditions,

(b) then, at least one (meth)acrylic monomer (II) is added, and

(c) as soon as 0 to 30% of the stoichiometry of the (meth)acrylic monomer(s) has been introduced into the reactor, the remainder of quaternising agent (III), the remainder of (meth)acrylic monomers and the water are added continuously and simultaneously until the desired concentration of unsaturated quaternary ammonium salts (I) has been obtained,

(d) and, in the case where the quaternising agent (III) is in the gaseous state at the reaction temperature, the reaction is carried out in the presence of oxygen and a pressure is applied in order that the quaternising agent should be in the liquid state and, at the end of reaction, the pressure is gradually decreased to atmospheric pressure and simultaneously a ratio of volumetric flow rate of total gas at the exit of the reactor to the volumetric flow rate of oxygen introduced into the reactor which is lower than 100 is applied.

2. Process according to Claim 1, characterised in that, in the case (d) a ratio of volumetric flow rate of total gas at the exit of the reactor to the volumetric flow rate of oxygen introduced into the reactor which is lower than 50 is applied.

3. Process according to Claim 1 or 2, characterised in that the temperature is kept between 30 and 60°C during the reaction.

4. Process according to any one of Claims 1 to 3, characterised in that the introduction of the remainder of quaternising agent (III), of the remainder of (meth)acrylic monomers (II) and of water in stage (c) is carried out as soon as 10 to 20 % of the stoichiometry of the (meth)acrylic monomer(s) has been introduced in accordance with stage (b).

5. Process according to Claim 1, characterised in that the reaction is carried out at a temperature of between 10 and 80°C, and preferably between 30 and 60°C, wherein,

($a_1$) in a first stage, all or a part of the liquid quaternising agent(s) (III) is introduced into the reactor,

($b_1$) then, at least one (meth)acrylic monomer (II) is added, and

($c_1$) as soon as 0 to 30 % of the stoichiometry of the (meth)acrylic monomer(s) has been introduced into the reactor, the remainder of quaternising agent (III) which is liquid at the reaction temperature, all or a part of the remainder of the methacrylic monomers (II) necessary for the reaction and all or a part of the water necessary for the reaction are added continuously and simultaneously, the total quantity of said quaternising agent (III) which is liquid at the reaction temperature representing 0.1 % to 99.9 % of the stoichiometry in relation to said (meth)acrylic monomers (II),

($d_1$) and then, the quaternising agent(s) (III) gaseous at the reaction temperature, the remainder of the (meth)acrylic monomers (II) and the remainder of water are added continuously and simultaneously until the desired concentration of unsaturated quaternary ammonium salts (I) is obtained, the sum of the quantities of quaternising agents added in stages ($a_1$), ($c_1$) and ($d_1$) corresponding to a value equal to or higher than the stoichiometry in relation to the (meth)acrylic monomers (II), the reaction being then carried out in the presence of oxygen at atmospheric pressure or at a higher pressure, and wherein, during the reaction, at the end of reaction and during the application of atmospheric pressure a ratio of volumetric flow rate of total gas at the exit of the reactor to the volumetric flow rate of oxygen introduced into the reactor which is lower than 100, and preferably lower than 50, is applied.